# FASCICULE DE BREVET EUROPEEN

(11) **EP 3 164 078 B1**
(45) Date de publication et mention de la délivrance du brevet: **01.01.2025**
(21) Numéro de dépôt: 15736571.9
(22) Date de dépôt: 29.06.2015
(51) Int. Cl.: G06T 7/00, A61B 8/08, G06T 7/149, G06T 7/174, G16H 50/30

(54) **PROCÉDÉ ET DISPOSITIF D'IMAGERIE FONCTIONNELLE DU CERVEAU**
VERFAHREN UND VORRICHTUNG ZUR FUNKTIONELLEN BILDGEBUNG DES GEHIRNS
METHOD AND DEVICE FOR FUNCTIONAL IMAGING OF THE BRAIN

(30) Priorité: 02.07.2014 FR 1456301
(43) Date de publication de la demande: 10.05.2017
(73) Titulaire: Centre National de la Recherche Scientifique (C.N.R.S.), 75016 Paris (FR); INSERM - Institut National de la Santé et de la Recherche Médicale, 75013 Paris (FR); Université Pierre et Marie Curie (Paris 6), 75005 Paris (FR); Ecole Supérieure de Physique et de Chimie Industrielles de la Ville de Paris, 75005 Paris (FR); Université Paris Cité, 75006 Paris (FR)
(72) Inventeur: TANTER, Mickaël, 92220 Bagneux (FR); GENNISSON, Jean-Luc, 95000 Cergy (FR); DEFFIEUX, Thomas, 94270 Kremlin Bicetre (FR); PERNOT, Mathieu, 75004 Paris (FR)
(74) Mandataire: Lavoix
(86) Numéro de dépôt international: PCT/FR2015/051752
(87) Numéro de publication internationale: WO 2016/001548

(56) Documents cités:
- US-A1- 2008 275 340
- US-A1- 2013 131 495
- EMILIE MACÉ ET AL: "Functional ultrasound imaging of the brain", NATURE METHODS, vol. 8, no. 8, 2 January 2011 (2011-01-02), pages 662 - 664, XP055015941, ISSN: 1548-7091, DOI: 10.1038/nmeth.1641
- MARTIJN E VAN RAAIJ ET AL: "Functional micro-ultrasound imaging of rodent cerebral hemodynamics", ULTRASONICS SYMPOSIUM (IUS), 2011 IEEE INTERNATIONAL, IEEE, 18 October 2011 (2011-10-18), pages 1258 - 1261, XP032230809, ISBN: 978-1-4577-1253-1, DOI: 10.1109/ULTSYM.2011.0310
- PENNEC X ET AL: "Tracking brain deformations in time sequences of 3D US images", PATTERN RECOGNITION LETTERS, ELSEVIER, AMSTERDAM, NL, vol. 24, no. 4-5, 2 February 2003 (2003-02-02), pages 801 - 813, XP004391218, ISSN: 0167-8655, DOI: 10.1016/S0167-8655(02)00183-6

## Description

### DOMAINE DE L'INVENTION

La présente invention est relative aux procédés et dispositifs d'imagerie fonctionnelle du cerveau.

### ARRIERE-PLAN DE L'INVENTION

L'imagerie fonctionnelle cérébrale consiste à imager les changements transitoires d'une zone du cerveau liée à une activité neuronale, chez l'animal ou l'être humain. Les zones du cerveau correspondant à différentes fonctions précises (par exemple déplacement de certains membres, utilisation de certains capteurs sensoriels, émotions, mémoire, etc.) sont répertoriées dans des atlas anatomiques fonctionnels connus, correspondant respectivement à diverses espèces animales ou à l'être humain (par exemple les atlas « Paxinos »), et l'imagerie fonctionnelle cérébrale permet de repérer et mesurer l'activité neuronale dans certaines de ces zones fonctionnelles, en correspondance avec une certaine activité de l'homme ou animal sur lequel est pratiquée l'imagerie fonctionnelle.

Certaines techniques d'imagerie fonctionnelle cérébrale sont basées sur la mesure des courants électriques (EEG) ou champs magnétiques (MEG) créés par les neurones. Ces techniques ont une résolution spatiale très limitée en raison de la complexité du problème inverse à résoudre pour retrouver la position des sources d'activité électriques.

Les autres techniques permettant d'imager ces changements fonctionnels sont basées sur le couplage neurovasculaire : lorsque les neurones ont une forte activité dans une zone du cerveau, un apport en glucose est nécessaire dans cette zone. Pour cela, le flux vasculaire augmente dans cette zone précise. En imageant les flux vasculaires dans le cerveau, on peut en déduire quelles sont les zones fonctionnelles activées dans l'atlas fonctionnel de l'homme ou animal dont le cerveau est imagé.

Plusieurs techniques sont utilisables pour imager ces changements de flux sanguins :
- l'IRM fonctionnelle (dite encore « IRMf ») qui image le changement d'oxygénation liée à un changement de flux sanguin,
- l'imagerie nucléaire (PET) qui image la fixation du glucose apporté par le flux sanguin,
- et l'imagerie fonctionnelle ultrasonore.

En IRMf, le repérage de la zone fonctionnelle activée se fait aisément, puisque les images IRM sont de très bonne qualité et permettent de repérer assez facilement l'anatomie et donc la zone fonctionnelle du cerveau activée. L'imagerie fonctionnelle est alors réalisée et superposée à une image anatomique réalisée par la machine IRMf avant l'acquisition de l'image fonctionnelle. Cette technologie présente toutefois de nombreux inconvénients : les machines IRMf sont très coûteuses et encombrantes, et n'offre une bonne résolution spatiale qu'au prix d'une baisse significative de la résolution temporelle, ce qui ne permet pas d'imager des phénomènes transitoires dans le cerveau (crise d'épilepsie par exemple).

Le PET a une très mauvaise résolution spatiale. Il donne une information fonctionnelle intéressante, mais une fois la zone activée repérée il n'est pas possible de savoir à quelle zone anatomique ou fonctionnelle du cerveau, elle correspond, de sorte qu'il est nécessaire de combiner une machine d'imagerie PET avec une machine IRM ou un scanner CT pour obtenir un résultat utilisable. Un tel ensemble d'imagerie est toutefois extrêmement coûteux.

L'imagerie fonctionnelle ultrasonore est fondée sur l'imagerie ultrasensible du flux sanguin (Macé et al, "Functional ultrasound imaging of the brain: theory and basic principles", IEEE Trans Ultrason Ferroelectr Freq Control. 2013 Mar;60(3):492-506), dont la variation au cours du temps donne accès aux zones fonctionnelles activées (Macé et al, "Functional ultrasound imaging of the brain", Nature Methods, 8, 662-664, 2011).

L'imagerie fonctionnelle ultrasonore permet d'obtenir, à relativement bas coût, une image vasculaire extrêmement précise du cerveau et la localisation des zones activées sur cette image vasculaire. Une difficulté est toutefois de pouvoir faire correspondre l'image vasculaire avec une image anatomique permettant de repérer les zones fonctionnelles. En effet, l'image anatomique que l'on peut acquérir en échographie par le dispositif d'imagerie fonctionnelle ultrasonore, est de trop faible qualité pour permettre de repérer les zones fonctionnelles imagées.

De ce fait, seul un opérateur expert est capable de repérer les zones fonctionnelles imagées en imagerie fonctionnelle ultrasonore : il doit repérer à l'œil dans l'image vasculaire, des structures globales du cerveau qui lui permettent de positionner l'image dans le cerveau, puis essayer de trouver où se trouve la zone fonctionnelle qui l'intéresse à l'aide d'un atlas.

Il est également connu des techniques d'imagerie des documents suivants :
- un article d'Emilie MACE et al. intitulé « Functional ultrasound imaging of the brain »*,*
- un article de Martijn E. VAN RAAIJ et al. intitulé « Functional micro-ultrasound imaging of rodent cérébral hemodynamics »*,*
- un article de X. PENNEC et al. intitulé « Tracking brain déformations in time sequences of 3D US images »*,*
- un document US 2013/131495 A1, et
- un document US 2008/275340.

### RESUME DE L'INVENTION

La présente invention a notamment pour but de pallier ces inconvénients.

A cet effet, l'invention propose un procédé d'imagerie fonctionnelle du cerveau par ultrasons selon la revendication 1.

Les atlas susmentionnés (atlas fonctionnel cérébral en correspondance avec l'atlas vasculaire cérébral permettent ainsi de repérer automatiquement, par reconnaissance de forme, quelles sont les zones fonctionnelles cérébrales visibles sur l'image vasculaire à étudier. L'identification des zones fonctionnelles du cerveau visualisées peut ainsi être effectuée aisément et rapidement, même par un utilisateur non expert. Les atlas fonctionnels et vasculaires correspondant aux différentes classes de sujets, peuvent être réalisées par avance et disponibles dans une bibliothèque d'atlas stockée sur ordinateur. Une classe de sujets peut par exemple correspondre à un ou plusieurs critères choisis notamment parmi : espèce, sexe, âge, poids.

Selon des modes de réalisation particuliers, le procédé peut également comporter une ou plusieurs des caractéristiques des revendications 2 à 14 lorsque ces caractéristiques sont techniquement compatible.

Dans divers modes de réalisation du procédé selon l'invention, on peut éventuellement avoir recours en outre à l'une et/ou à l'autre des dispositions suivantes :
- l'image vasculaire à étudier est représentative des écoulements sanguins dans au moins une partie du cerveau ;
- l'image vasculaire à étudier comporte au moins une information hémodynamique choisie parmi : le volume vasculaire cérébral (cf (Macé et al, "Functional ultrasound imaging of the brain: theory and basic principles", IEEE Trans Ultrason Ferroelectr Freq Control. 2013 Mar; 60 (3) :492-506), le Doppler puissance, la vitesse d'écoulement du sang, le Doppler couleur, une valeur représentative de la résistance circulatoire (indice de Pourcelot ou autre) ;

- la cartographie vasculaire fonctionnelle de référence est une cartographie tridimensionnelle ; par exemple elle peut comprendre une pluralité de coupes cérébrales dans des plans successifs ;
- chaque image vasculaire initiale est tridimensionnelle ;
- chaque image vasculaire initiale est bidimensionnelle, la cartographie vasculaire fonctionnelle de référence étant obtenue à partir d'images vasculaires de référence réalisées selon une pluralité de plans successifs ;
   - on applique au sujet un stimulus et on détecte sur l'image vasculaire cérébrale (IVO) une activation d'au moins une zone fonctionnelle (1c) suite à ce stimulus ;
- on visualise sur l'image vasculaire cérébrale une action ciblée vers au moins une zone fonctionnelle identifiée sur l'image vasculaire cérébrale, et on guide cette action vers ladite zone fonctionnelle identifiée ;
- ladite action est choisie parmi une implantation d'électrode, une injection de fluide, une stimulation à distance par une onde (électromagnétique, sonore, ultrasonore, lumineuse) ;
- l'étape d'imagerie est réalisée avec au moins une sonde ultrasonore déplaçable par des moyens motorisés, et on déplace ladite au moins une sonde jusqu'à ce que l'image vasculaire cérébrales corresponde à au moins une zone fonctionnelle souhaitée ;
- l'étape d'imagerie (a) est réalisée avec au moins une sonde ultrasonore adaptée pour réaliser une image tridimensionnelle, et on extrait de cette image tridimensionnelle une image bidimensionnelle correspondant à au moins une zone fonctionnelle souhaitée.

Par ailleurs, l'invention a également pour objet un dispositif selon la revendication 15.

### BREVE DESCRIPTION DES DESSINS

D'autres caractéristiques et avantages de l'invention apparaîtront au cours de la description suivante d'un de ses modes de réalisation, donné à titre d'exemple non limitatif, en regard des dessins joints.

Sur les dessins :
- la figure 1 est une vue schématique d'un dispositif pour la mise en oeuvre d'un procédé selon un mode de réalisation de l'invention,
- la figure 2 est un schéma bloc d'une partie du dispositif de la figure 1,
- la figure 3 est une vue en perspective d'un cerveau de rat montrant un plan d'imagerie dans le cas d'une imagerie bidimensionnelle et un plan de coronal de référence,
- la figure 4 est un exemple d'image vasculaire à étudier pouvant être réalisée par imagerie ultrasonore par exemple à partir du cerveau de la figure 3, dans un plan coronal;
- la figure 5 est un exemple de carte fonctionnelle cérébrale pouvant correspondre par exemple au cerveau de la figure 3, dans un plan coronal ;
- la figure 6 montre un ensemble d'images vasculaires initiales prises dans des plans coronaux successifs sur un cerveau de rat en vue de participer à la constitution d'un atlas vasculaire cérébral d'une certaine classe de rats ;
- la figure 7 illustre schématiquement la réalisation dudit atlas vasculaire cérébral ;
- la figure 8 illustre schématiquement la réalisation d'une image vasculaire cérébrale coronale incorporant une carte fonctionnelle selon l'invention, en utilisant la seule imagerie ultrasonore, et
- la figure 9 montre un exemple d'images vasculaires fonctionnelles successives obtenues par le procédé de l'invention, permettant de suivre en temps réel l'activation de certaines zones fonctionnelles du cerveau.

### DESCRIPTION DETAILLEE

Sur les différentes figures, les mêmes références désignent des éléments identiques ou similaires.

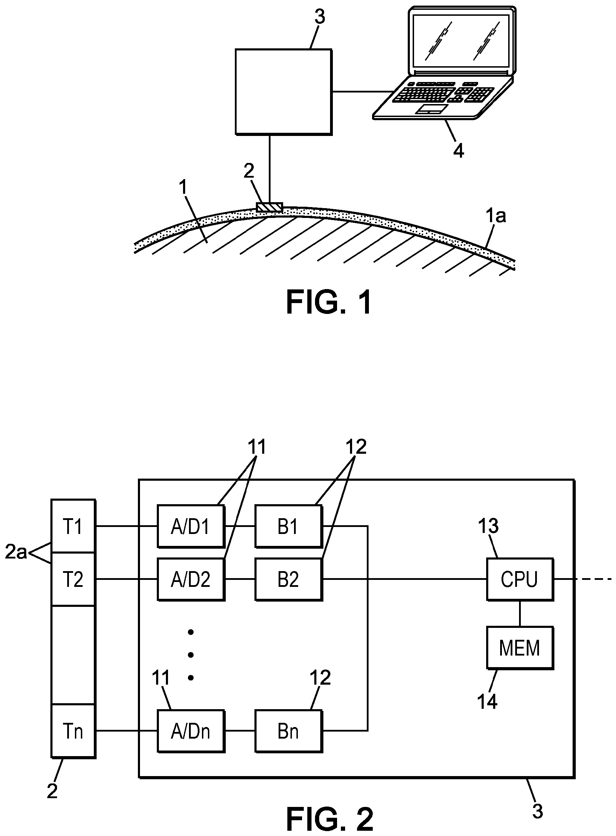

La figure 1 montre un exemple de dispositif adapté pour réaliser une imagerie vasculaire du cerveau 1 d'un sujet humain ou animal à étudier, par émission et réception d'ondes ultrasonores de compression, par exemple de fréquences comprises entre 1 et 40 MHz. L'émission et réception d'ondes ultrasonores peut se faire au moyen d'un réseau 2 de transducteurs ultrasonores soit en traversant tout ou partie du crâne 1a du sujet, soit directement au contact du cerveau 1, notamment au niveau d'une ou plusieurs trépanations du crâne 1a.

Le dispositif d'imagerie comporte par exemple, comme illustré sur les figures 1 et 2 :
- un réseau 2 de n transducteurs ultrasonores 2a (T₁-Tₙ), comprenant par exemple quelques centaines de transducteurs 2a, ce réseau 2 pouvant être par exemple une barrette de transducteurs (réseau 1D) adapté pour réaliser une image bidimensionnelle (2D) ou encore un réseau à deux dimensions adapté pour réaliser une image tridimensionnelle (3D) ;
- un circuit électronique 3 commandant le réseau de transducteurs 2 et adapté pour faire émettre des ondes ultrasonores par le réseau de transducteurs et acquérir les signaux captés par ce réseau de transducteurs ;
- un ordinateur 4 ou similaire pour commander le circuit électronique 3 et visualiser les images ultrasonores obtenues à partir desdits signaux captés.

Comme représenté sur la figure 2, le circuit électronique 3 peut comprendre par exemple :
- n convertisseurs analogique / digital 11 (A/Dᵢ) connectés individuellement aux n transducteurs T₁-T_{N} du réseau 2 de transducteurs,
- n mémoires tampon 12 (Bᵢ) respectivement connectées aux n convertisseurs analogique / digital 11,
- une unité centrale 13 (CPU) communiquant avec les mémoires tampon 12 et l'ordinateur 4 et adapté au traitement des signaux envoyés vers le réseau de transducteurs 2 et reçus dudit réseau de transducteurs,
- une mémoire 14 (MEM) connectée à l'unité centrale 13.

Le dispositif d'imagerie représenté sur les figures 1 et 2 est adapté pour réaliser une imagerie ultrasonore synthétique du cerveau permettant d'imager finement le réseau vasculaire cérébral et de donner au moins une information hémodynamique (c'est-à-dire relative aux flux sanguins), comme décrit notamment par Macé et al. (publications susmentionnées "Functional ultrasound imaging of the brain: theory and basic principles", IEEE Trans Ultrason Ferroelectr Freq Control. 2013 Mar; 60 (3):492 - 506 ) et "Functional ultrasound imaging of the brain", Nature Methods, 8, 662-664, 2011) et dans le document EP2101191.

L'information hémodynamique donnée par l'image vasculaire cérébrale ainsi obtenue peut être choisie notamment parmi : le volume vasculaire cérébral, le Doppler puissance, la vitesse d'écoulement du sang, le Doppler couleur, une valeur représentative de la résistance circulatoire (indice de Pourcelot ou autre).

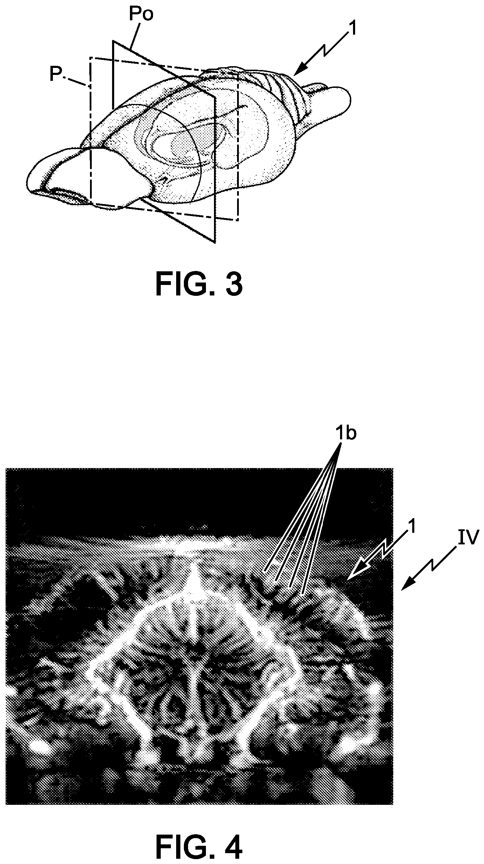

Comme représenté sur la figure 3, dans le cas de l'imagerie bidimensionnelle, l'image vasculaire cérébrale peut être réalisée dans un plan P, par exemple de direction proche d'un plan coronal P0 mais en pratique le plus souvent différent du plan coronal parfait P0. L'image vasculaire cérébrale IV obtenue, visible sur la figure 4, permet de visualiser les vaisseaux sanguins 1b du cerveau 1 avec une excellente définition, permettant de visualiser y compris des micro-vaisseaux. L'information hémodynamique susmentionnée peut être donnée par l'intensité lumineuse de chaque pixel et / ou par une échelle de couleurs.

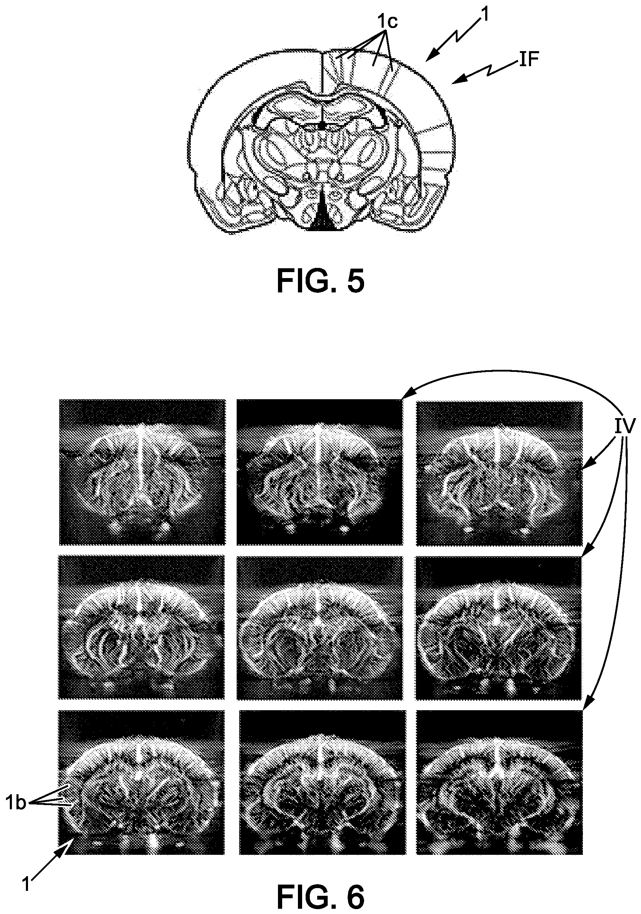

Cette information hémodynamique reflète l'activité cérébrale locale et permet donc de faire de l'imagerie fonctionnelle, à condition de pouvoir repérer chaque pixel de l'image dans un atlas anatomique fonctionnel du cerveau (Paxinos ou autre) dont une coupe coronale IF est représentée à titre d'exemple sur la figure 5. Un tel atlas anatomique fonctionnel contient un atlas anatomique type du cerveau (forme des tissus cérébraux) et un atlas fonctionnel type constitué de zones fonctionnelles 1c repérées sur cet atlas anatomique type. Chacune des zones fonctionnelles 1c de l'atlas fonctionnel type correspond à une fonction motrice, sensitive ou cognitive précise et porte une référence déterminée correspondant à cette fonction. Chaque atlas anatomique fonctionnel est valable pour une classe de sujets étudiés, correspondant par exemple à une espèce, un sexe (masculin / féminin), une plage de poids et une plage d'âge du sujet étudié (ou seulement certains de ces critères, plus d'autres éventuellement).

Il est toutefois très difficile pour un opérateur de repérer l'image vasculaire cérébrale IV dans l'atlas anatomique fonctionnel, puisque d'une part, l'image vasculaire cérébrale IV ne correspond pas aux images anatomiques pouvant être extraits de l'atlas anatomique type et d'autre part, le plan P de l'image vasculaire cérébrale n'est généralement pas exactement un plan coronal P0 comme expliqué ci-dessus.

Pour remédier à cet inconvénient, l'invention prévoit de réaliser l'avance, pour au moins certaines classes de sujets à étudier, un atlas vasculaire cérébral AV type correspondant à ladite classe de sujets, en correspondance avec un atlas fonctionnel cérébral type AF correspondant audit atlas vasculaire cérébral AV et dont les zones fonctionnelles cérébrales 1c sont repérées dans cet atlas vasculaire cérébral AV.

Ces deux atlas peuvent être établis par avance notamment au cours d'une étape préliminaire (p) de cartographie vasculaire fonctionnelle de référence, comprenant les sous-étapes suivantes :
(p1) une sous étape d'imagerie de référence au cours de laquelle on réalise à la fois :
   - ledit atlas vasculaire cérébral (réalisé par imagerie ultrasonore vasculaire du cerveau, ou encore par angioscanner, IRM, CT scanner) d'au moins un sujet de la classe de sujets considérée,
   - et un atlas anatomique de référence réalisé en imageant le cerveau dudit au moins un sujet de la classe de sujets considérée, par un deuxième type imagerie donnant une image anatomique du cerveau plus précise que l'imagerie ultrasonore, par exemple par IRM,
(p2) une sous-étape de cartographie fonctionnelle de référence au cours de laquelle on détermine ledit atlas fonctionnel cérébral à partir d'un atlas anatomique fonctionnel (Paxinos ou autre) qui comprend au moins un atlas anatomique type du cerveau et des zones fonctionnelles repérées sur cet atlas anatomique type, cette sous-étape de cartographie fonctionnelle de référence étant réalisée en faisant correspondre ledit atlas anatomique de référence avec l'atlas anatomique type, pour repérer les zones fonctionnelles de l'atlas anatomique fonctionnel sur ledit atlas vasculaire cérébral.

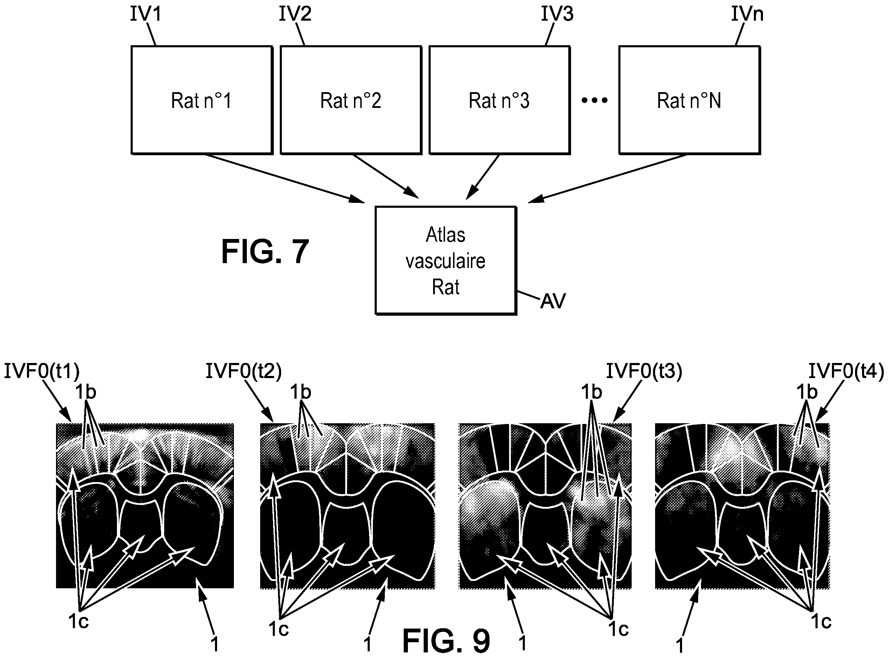

Au cours de ladite sous étape (p1) d'imagerie de référence, on peut réaliser successivement sur plusieurs sujets de la classe de sujets considérée, respectivement plusieurs images vasculaires initiales IV (figure 6) par imagerie ultrasonore vasculaire du cerveau et plusieurs images anatomiques initiales par ledit deuxième type imagerie. On détermine ledit atlas vasculaire cérébral et ledit atlas anatomique de référence par calcul statistique respectivement à partir desdites images vasculaires initiales et des images anatomiques initiales (par exemple, respectivement par moyenne desdites images vasculaires initiales et des images anatomiques initiales), comme illustré schématiquement sur la figure 7 pour l'atlas vasculaire cérébral chez le rat.

Au cours de la sous-étape (p2) de cartographie fonctionnelle de référence, on peut faire correspondre l'atlas anatomique de référence avec l'atlas anatomique type par reconnaissance de forme. Par exemple, on détermine une déformation géométrique permettant de passer dudit atlas anatomique de référence à l'atlas anatomique type, puis on applique cette déformation géométrique à l'atlas vasculaire cérébral et on met ainsi en correspondance l'atlas vasculaire cérébral avec les zones fonctionnelles de l'atlas anatomique fonctionnel.

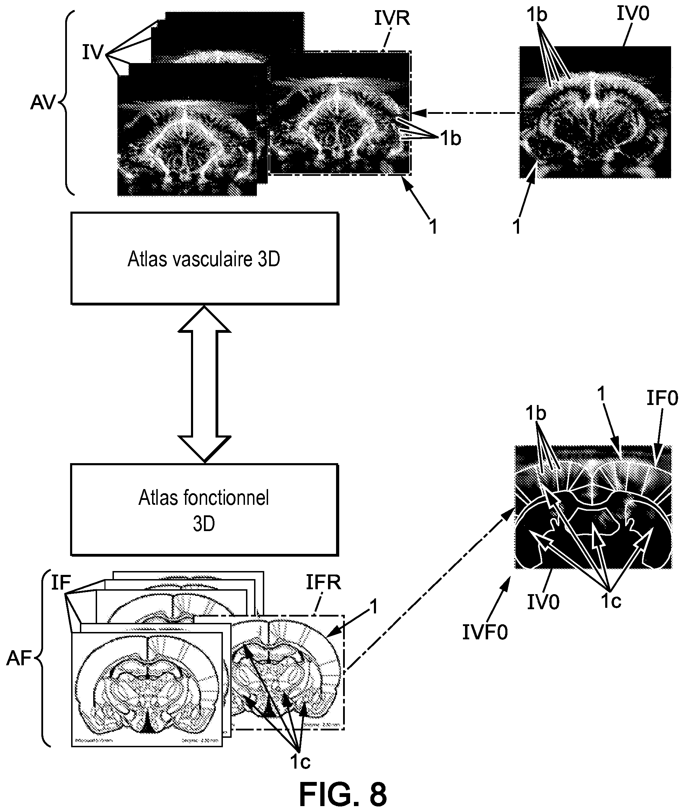

Une fois établi l'atlas vasculaire cérébral AV type correspondant à une classe de sujets, en correspondance avec l'atlas fonctionnel cérébral AF type pour la même classe de sujets, on peut aisément et automatiquement repérer les zones fonctionnelles sur une image vasculaire cérébrale IVO réalisée uniquement par imagerie ultrasonore, par le processus illustré sur la figure 8 :

### (a) Imagerie :

On image un cerveau 1 du sujet par imagerie ultrasonore, pour obtenir l'image vasculaire à étudier IV0.

### (b0) Repérage préliminaire éventuel:

Eventuellement, on effectue d'abord un repérage préliminaire grossier au cours duquel on détecte automatiquement sur l'image vasculaire à étudier IV0, au moins une zone caractéristique normalement présente chez tout sujet à étudier et dans l'atlas vasculaire cérébral AV, et on situe ainsi grossièrement l'image vasculaire à étudier IVO dans l'atlas vasculaire cérébral AV. Ladite zone caractéristique peut être un point ou une structure invariant(e) du cerveau, par exemple le polygone de Willis, les veines sylviennes, l'artère cérébrale antérieure, les plus grosses artères du cerveau.

### (b) Repérage :

Par reconnaissance de forme, on compare automatiquement par corrélation, l'image vasculaire à étudier IVO avec l'atlas vasculaire cérébral AV correspondant à la classe de sujets à laquelle appartient le sujet étudié, et on situe ainsi l'image vasculaire à étudier IVO dans l'atlas vasculaire cérébral AV. En pratique, cette étape de repérage de l'image vasculaire à étudier IVO dans l'atlas vasculaire AV se fait en on déterminant par reconnaissance de forme, une image vasculaire IVR issue de l'atlas vasculaire AV, correspondant le mieux à l'image vasculaire à étudier IV0. L'atlas vasculaire AV peut éventuellement être constitué par une série d'images vasculaires cérébrales IV bidimensionnelles prises dans des plans adjacents successifs (par exemple des plans coronaux, ou autres), et le repérage susmentionné peut consister, dans une version simplifiée, dans le choix de l'image vasculaire cérébrale IVR la plus proche de l'image vasculaire à étudier IVO (qui dans ce cas est une image bidimensionnelle).Dans une version plus élaborée et lorsque l'image vasculaire à étudier IVO est bidimensionnelle, ce repérage consiste également à déterminer par reconnaissance de forme, l'orientation et la position du plan de l'image IV0, et à reconstituer une image IVR issue de l'atlas vasculaire AV dans ce plan (que l'atlas soit tridimensionnel ou constitué par une série d'images vasculaires cérébrales IV bidimensionnelles prises dans des plans adjacents successifs).

### (c) Identification :

A l'aide de l'atlas fonctionnel cérébral AF correspondant audit atlas vasculaire cérébral AV, on identifie au moins une zone fonctionnelle cérébrale 1c sur l'image vasculaire à étudier IV0. Plus précisément, on détermine une image IFR issue de l'atlas fonctionnel AF, correspondant à l'image IVR susmentionnée, et on met en correspondance cette image IFR avec l'image vasculaire à étudier IV0, par déformation géométrique, pour déterminer ainsi une carte fonctionnelle IFO (formée par les limites des zones fonctionnelles 1c), que l'on superpose à l'image vasculaire à étudier IVO pour obtenir ainsi une image vasculaire fonctionnelle IVF0 du cerveau 1.

Plus précisément, au cours de l'étape de repérage (b), on peut déterminer une déformation géométrique permettant de passer de l'image IVR à l'image vasculaire à étudier IV0, et au cours de l'étape d'identification (c), on peut déterminer la carte fonctionnelle IFO de l'image vasculaire à étudier IVO en appliquant cette déformation géométrique à l'image IFR issue de de l'atlas fonctionnel cérébral AF.

Une fois la carte fonctionnelle IFO établie, elle reste inchangée tant que la sonde d'imagerie ultrasonore 2 reste immobile (cette sonde peut par exemple être fixée de façon rigide au crâne 1a du sujet, ou par l'intermédiaire d'un actionneur motorisé permettant de commander des déplacements contrôlés de cette sonde). On peut alors réaliser des images vasculaires cérébrales IVO au cours du temps (voir figure 9), par exemple en synchronisme avec des stimulations externes ou internes appliquées au sujet à étudier (y compris activité cérébrale consciente ou inconsciente). Ces images vasculaires cérébrales sont superposées à la carte fonctionnelle IF0, de façon à visualiser et éventuellement enregistrer l'activation des zones fonctionnelles 1c dans le temps.

On peut ainsi également visualiser et guider l'implantation d'électrodes ou l'injection d'agents thérapeutiques particuliers vers des zones fonctionnelles ciblées, ou encore une stimulation à distance par une onde (électromagnétique, ultrasonore, sonore ou lumineuse).

On notera également que le procédé décrit ci-dessus permet aisément de déplacer la sonde ultrasonore 2 vers une position souhaitée pour imager certaines zones fonctionnelles définies, lorsque ladite sonde 2 fixée de façon rigide au crâne 1a du sujet, ou par l'intermédiaire d'un actionneur motorisé permettant de commander des déplacements contrôlés de cette sonde. En effet, l'image vasculaire cérébrale IVO étant repérée dans l'atlas fonctionnel AF, il est aisé de déterminer le déplacement nécessaire pour visualiser une zone fonctionnelle 1c souhaitée.

De même, lorsque l'imagerie ultrasonore est réalisée avec au moins une sonde ultrasonore 2 adaptée pour réaliser une image tridimensionnelle, on peut extraire de cette image tridimensionnelle une image bidimensionnelle correspondant à au moins une zone fonctionnelle souhaitée 1c.

## Revendications

1. Procédé d'imagerie fonctionnelle du cerveau par ultrasons, comprenant les étapes suivantes :
(a) une étape d'imagerie vasculaire au cours de laquelle on image un cerveau (1) d'un sujet humain ou animal par imagerie ultrasonore, pour obtenir une image vasculaire à étudier (IVO), l'image vasculaire à étudier (IVO) est représentative des écoulements sanguins dans au moins une partie du cerveau, ledit sujet appartenant à une certaine classe de sujets,
(b) une étape de repérage, au cours de laquelle, par reconnaissance de forme, on compare automatiquement au moins une partie de l'image vasculaire à étudier (IVO) avec un atlas vasculaire cérébral (AV) correspondant à ladite classe de sujets, et on situe ainsi l'image vasculaire à étudier (IVO) dans l'atlas vasculaire cérébral (AV),
(c) une étape d'identification au cours de laquelle on utilise un atlas fonctionnel cérébral (AF) correspondant audit atlas vasculaire cérébral (AV) et comprenant des zones fonctionnelles cérébrales (1c) repérées dans cet atlas vasculaire cérébral (AV), de sorte que l'on identifie au moins une zone fonctionnelle cérébrale (1c) sur l'image vasculaire à étudier (IV0).

2. Procédé selon la revendication 1, dans lequel au cours de l'étape de repérage (b), on met en correspondance une partie (IVR) de l'atlas vasculaire cérébral (AV) avec l'image vasculaire à étudier (IVO), et au cours de l'étape d'identification, on détermine une carte de zones fonctionnelles (IF0) correspondant à ladite partie de l'atlas vasculaire cérébral (AV), et on superpose l'image vasculaire à étudier (IVO) avec ladite carte de zones fonctionnelles (IF0).

3. Procédé selon la revendication 2, dans lequel, au cours de l'étape de repérage, on détermine une déformation géométrique permettant de passer de ladite au moins une partie (IVR) de l'atlas vasculaire cérébral (AF) à l'image vasculaire à étudier, et au cours de l'étape d'identification, on détermine la carte fonctionnelle (IF0) de l'image vasculaire à étudier en appliquant cette déformation géométrique à une partie (IFR) de l'atlas fonctionnel cérébral (AF) correspondant à ladite partie (IVR) de l'atlas vasculaire cérébral (AV) qui correspond à l'image vasculaire à étudier (IV0).

4. Procédé selon l'une quelconque des revendications 1 à 3, dans lequel à l'étape de repérage (b), ladite reconnaissance de forme est effectuée par corrélation entre l'image vasculaire à étudier (IVO) et l'atlas vasculaire cérébral (AV).

5. Procédé selon l'une quelconque des revendications 1 à 4, dans lequel l'image vasculaire à étudier (IVO) comporte au moins une information hémodynamique choisie parmi : le volume vasculaire cérébral, le Doppler puissance, la vitesse d'écoulement du sang, le Doppler couleur, une valeur représentative de la résistance circulatoire.

6. Procédé selon l'une quelconque des revendications 1 à 5, dans lequel l'atlas vasculaire cérébral contient des données issues d'une imagerie choisie parmi : l'imagerie ultrasonore, l'angioscanner, l'IRM, le CT scanner.

7. Procédé selon l'une quelconque des revendications 1 à 6, comprenant en outre, entre l'étape d'imagerie (a) et l'étape de repérage (b), une étape de repérage préliminaire (b0) au cours de laquelle on détecte automatiquement sur l'image vasculaire à étudier (IVO), au moins une zone caractéristique normalement présente chez tout sujet à étudier et dans l'atlas vasculaire cérébral (AV), et on situe ainsi grossièrement l'image vasculaire à étudier (IVO) dans l'atlas vasculaire cérébral (AV).

8. Procédé selon la revendication 7, dans lequel ladite zone caractéristique est choisie parmi : le polygone de Willis, les veines sylviennes, l'artère cérébrale antérieure, les plus grosses artères du cerveau.

9. Procédé selon l'une quelconque des revendications 1 à 8, comportant au moins une étape préliminaire (p) de cartographie vasculaire fonctionnelle de référence, comprenant les sous-étapes suivantes :
(p1) une sous étape d'imagerie de référence au cours de laquelle on réalise à la fois :
- ledit atlas vasculaire cérébral (AV) d'au moins un sujet de la classe de sujets considérée,
- et un atlas anatomique de référence réalisé en imageant le cerveau dudit moins un sujet de la classe de sujets considérée, par un deuxième type d'imagerie donnant une image anatomique du cerveau plus précise que l'imagerie ultrasonore,
(p2) une sous-étape de cartographie fonctionnelle de référence au cours de laquelle on détermine ledit atlas fonctionnel cérébral (AF) à partir d'un atlas anatomique fonctionnel qui comprend au moins un atlas anatomique type du cerveau et des zones fonctionnelles repérées sur cet atlas anatomique type, cette sous-étape de cartographie fonctionnelle de référence étant réalisée en faisant correspondre ledit atlas anatomique de référence avec l'atlas anatomique type, pour repérer les zones fonctionnelles de l'atlas anatomique fonctionnel sur ledit atlas vasculaire cérébral.

10. Procédé selon la revendication 9, dans lequel au cours de la sous-étape (p1) d'imagerie de référence, on réalise ledit atlas vasculaire cérébral (AV) par imagerie ultrasonore vasculaire du cerveau.

11. Procédé selon la revendication 9 ou la revendication 10, dans lequel au cours de la sous-étape (p2) de cartographie fonctionnelle de référence, on fait correspondre l'atlas anatomique de référence avec l'atlas anatomique type par reconnaissance de forme.

12. Procédé selon la revendication 11, dans lequel au cours de la sous-étape (p2) de cartographie de référence, on détermine une déformation géométrique permettant de passer dudit atlas anatomique de référence à l'atlas anatomique type, on applique cette déformation géométrique à l'atlas vasculaire cérébral (AV) et on met ainsi en correspondance l'atlas vasculaire cérébral (AV) avec les zones fonctionnelles de l'atlas anatomique fonctionnel.

13. Procédé selon l'une quelconque des revendications 9 à 12, dans lequel au cours de ladite sous étape (p1) d'imagerie de référence, on réalise successivement sur plusieurs sujets de la classe de sujets considérée, respectivement plusieurs images vasculaires (IV) initiales par imagerie ultrasonore vasculaire du cerveau et plusieurs images anatomiques initiales par ledit deuxième type imagerie, et on détermine ledit atlas vasculaire cérébral (AV) et ledit atlas anatomique de référence par calcul statistique respectivement à partir desdites images vasculaires initiales et des images anatomiques initiales.

14. Procédé selon l'une quelconque des revendications 1 à 13, dans lequel chaque classe de sujets correspond à au moins un critère choisi parmi : espèce, sexe, âge, poids.

15. Dispositif pour la mise en oeuvre d'un procédé selon l'une quelconque des revendications 1 à 14, comprenant :
- des moyens d'imagerie vasculaire (2-4) adaptés pour imager un cerveau d'un sujet humain ou animal par imagerie ultrasonore, pour obtenir une image vasculaire à étudier (IVO), ledit sujet appartenant à une certaine classe de sujets,
- des moyens de repérage (4) par reconnaissance de forme, adaptés pour comparer automatiquement l'image vasculaire à étudier (IVO) avec un atlas vasculaire cérébral (AV) correspondant à ladite classe de sujets, et pour situer ainsi l'image vasculaire à étudier (IVO) dans l'atlas vasculaire cérébral (AV),
- des moyens d'identification (4) utilisant un atlas fonctionnel cérébral (AF) correspondant audit atlas vasculaire cérébral (AV) et comprenant des zones fonctionnelles cérébrales (1c) repérées dans cet atlas vasculaire cérébral (AV), lesdits moyens d'identification étant adaptés pour identifier au moins une zone fonctionnelle cérébrale (1c) sur l'image vasculaire à étudier (IV0).

## Patentansprüche

1. Verfahren zur funktionellen Bildgebung des Gehirns durch Ultraschall, das die folgenden Schritte umfasst:
(a) einen Schritt der Gefäßbildgebung, bei dem ein Gehirn (1) eines menschlichen oder tierischen Subjekts durch Ultraschallbildgebung abgebildet wird, um ein zu untersuchendes Gefäßbild (IVO) zu erhalten, wobei das zu untersuchende Gefäßbild (IVO) für die Blutflüsse in mindestens einem Teil des Gehirns repräsentativ ist, wobei das Subjekt zu einer bestimmten Subjektklasse gehört,
(b) einen Lokalisierungsschritt, bei dem durch Mustererkennung automatisch mindestens ein Teil des zu untersuchenden Gefäßbildes (IVO) mit einem Hirngefäßatlas (AV) verglichen wird, der der Subjektklasse entspricht, und dadurch das zu untersuchende Gefäßbild (IVO) in dem Hirngefäßatlas (AV) lokalisiert wird,
(c) einen Identifikationsschritt, bei dem ein Hirnfunktionsatlas (AF) verwendet wird, der dem Hirngefäßatlas (AV) entspricht und Hirnfunktionsbereiche umfasst (1c), die in diesem Hirngefäßatlas (AV) lokalisiert sind, so dass mindestens ein Hirnfunktionsbereich (1c) auf dem zu untersuchenden Gefäßbild (IVO) identifiziert wird.

2. Verfahren nach Anspruch 1, wobei bei dem Lokalisierungsschritt (b) ein Teil (IVR) des Hirngefäßatlas (AV) mit dem zu untersuchenden Gefäßbild (IVO) in Übereinstimmung gebracht wird, und bei dem Identifikationsschritt eine Funktionsbereichskarte (IF0) bestimmt wird, die dem Teil des Hirngefäßatlas (AV) entspricht, und das zu untersuchende Gefäßbild (IVO) mit der Funktionsbereichskarte (IF0) überlagert wird.

3. Verfahren nach Anspruch 2, wobei bei dem Lokalisierungsschritt eine geometrische Verzerrung bestimmt wird, die es ermöglicht, von dem mindestens einen Teil (IVR) des Hirngefäßatlas (AF) zu dem zu untersuchenden Gefäßbild zu gelangen, und bei dem Identifikationsschritt die Funktionskarte (IF0) des zu untersuchenden Gefäßbildes bestimmt wird, indem diese geometrische Verzerrung auf einen Teil (IFR) des Hirnfunktionsatlas (AF) angewendet wird, der dem Teil (IVR) des Hirngefäßatlas (AV) entspricht, der dem zu untersuchenden Gefäßbild (IVO) entspricht.

4. Verfahren nach einem der Ansprüche 1 bis 3, wobei im Lokalisierungsschritt (b) die Mustererkennung durch Korrelation zwischen dem zu untersuchenden Gefäßbild (IVO) und dem Hirngefäßatlas (AV) durchgeführt wird.

5. Verfahren nach einem der Ansprüche 1 bis 4, wobei das zu untersuchende Gefäßbild (IVO) mindestens eine hämodynamische Information enthält, die aus dem Hirngefäßvolumen, dem Power-Doppler, der Blutströmungsgeschwindigkeit, dem Farb-Doppler, einem Wert, der für den Kreislaufwiderstand repräsentativ ist, ausgewählt ist.

6. Verfahren nach einem der Ansprüche 1 bis 5, wobei der Hirngefäßatlas Daten aus einer Bildgebung enthält, die aus Ultraschallbildgebung, Angioscanner, MRT, CT-Scanner ausgewählt ist.

7. Verfahren nach einem der Ansprüche 1 bis 6, das zwischen dem Bildgebungsschritt (a) und dem Lokalisierungsschritt (b) ferner einen vorbereitenden Lokalisierungsschritt (b0) umfasst, bei dem auf dem zu untersuchenden Gefäßbild (IVO) mindestens ein charakteristischer Bereich, der normalerweise bei jedem zu untersuchenden Subjekt und im Hirngefäßatlas (AV) vorhanden ist, automatisch erkannt wird und somit das zu untersuchende Gefäßbild (IVO) grob im Hirngefäßatlas (AV) verortet wird.

8. Verfahren nach Anspruch 7, wobei der charakteristische Bereich aus dem Willis-Polygon, den Sylvischen Venen, der vorderen Hirnarterie, den größten Arterien des Gehirns ausgewählt ist.

9. Verfahren nach einem der Ansprüche 1 bis 8, das mindestens einen vorbereitenden Schritt (p) der funktionellen Referenzgefäßkartierung aufweist, der die folgenden Unterschritte umfasst:
(p1) einen Unterschritt der Referenzbildgebung, bei dem sowohl als auch erstellt wird:
- der Hirngefäßatlas (AV) von mindestens einem Subjekt der betrachteten Subjektklasse,
- und ein anatomischer Referenzatlas, der erstellt wird, indem das Gehirn des mindestens einen Subjekts aus der betrachteten Subjektklasse durch eine zweite Art der Bildgebung abgebildet wird, die ein anatomisches Bild des Gehirns liefert, das genauer ist als die Ultraschallbildgebung,
(p2) einen Unterschritt der funktionellen Referenzkartierung, bei dem der Hirnfunktionsatlas (AF) ausgehend von einem anatomischen Funktionsatlas bestimmt wird, der mindestens einen typischen anatomischen Atlas des Gehirns und der Funktionsbereiche umfasst, die in diesem typischen anatomischen Atlas lokalisiert sind, wobei dieser Unterschritt der funktionellen Referenzkartierung durchgeführt wird, indem der anatomische Referenzatlas mit dem typischen anatomischen Atlas abgeglichen wird, um die Funktionsbereiche des anatomischen Funktionsatlas auf dem Hirngefäßatlas zu lokalisieren.

10. Verfahren nach Anspruch 9, wobei bei dem Unterschritt (p1) der Referenzbildgebung der Hirngefäßatlas (AV) durch vaskuläre Ultraschallbildgebung des Gehirns erstellt wird.

11. Verfahren nach Anspruch 9 oder Anspruch 10, wobei bei dem Unterschritt (p2) der funktionellen Referenzkartierung der anatomische Referenzatlas mit dem typischen anatomischen Atlas durch Mustererkennung abgeglichen wird.

12. Verfahren nach Anspruch 11, wobei bei dem Unterschritt (p2) der Referenzkartierung eine geometrische Verzerrung bestimmt wird, die es ermöglicht, vom anatomischen Referenzatlas zum typischen anatomischen Atlas zu gelangen, diese geometrische Verzerrung auf den Hirngefäßatlas (AV) angewendet wird und so der Hirngefäßatlas (AV) mit den Funktionsbereichen des anatomischen Funktionsatlas in Übereinstimmung gebracht wird.

13. Verfahren nach einem der Ansprüche 9 bis 12, wobei bei dem Unterschritt (p1) der Referenzbildgebung nacheinander an mehreren Subjekten der betrachteten Subjektklasse jeweils mehrere Ausgangs-Gefäßbilder (IV) durch vaskuläre Ultraschall-Bildgebung des Gehirns und mehrere anatomische Ausgangs-Bilder durch die zweite Art der Bildgebung aufgenommen werden und der Hirngefäßatlas (AV) und der anatomischen Referenzatlas durch statistische Berechnung jeweils ausgehend von den Ausgangs-Gefäßbildern und den anatomischen Ausgangs-Bildern bestimmt wird.

14. Verfahren nach einem der Ansprüche 1 bis 13, wobei jede Subjektklasse mindestens einem Kriterium entspricht, das aus Spezies, Geschlecht, Alter, Gewicht ausgewählt ist.

15. Vorrichtung für die Durchführung eines Verfahrens nach einem der Ansprüche 1 bis 14, umfassend:
- Gefäßbildgebungsmittel (2-4), die geeignet sind, ein Gehirn eines menschlichen oder tierischen Subjekts durch Ultraschallbildgebung abzubilden, um ein zu untersuchendes Gefäßbild (IVO) zu erhalten, wobei das Subjekt zu einer bestimmten Subjektklasse gehört,
- Lokalisierungsmittel (4) durch Mustererkennung, die geeignet sind, das zu untersuchende Gefäßbild (IVO) automatisch mit einem Hirngefäßatlas (AV) zu vergleichen, der der Subjektklasse entspricht, und somit das zu untersuchende Gefäßbild (IVO) in dem Hirngefäßatlas (AV) zu lokalisieren,
- Identifikationsmittel (4), die einen Hirnfunktionsatlas (AF) verwenden, der dem Hirngefäßatlas (AV) entspricht und Hirnfunktionsbereiche (1c) umfasst, die in dem Hirngefäßatlas (AV) lokalisiert sind, wobei die Identifikationsmittel geeignet sind, mindestens einen Hirnfunktionsbereich (1c) auf dem zu untersuchenden Gefäßbild (IVO) zu identifizieren.

## Claims

1. A method for functional imaging of the brain by ultrasonic waves, comprising the following steps:
(a) a vascular imaging step during which a brain (1) of a human or animal subject is imaged by ultrasonic imaging, in order to obtain a vascular image to be studied (VI0), the vascular image to be studied being representative of the blood flows in at least one portion of the brain, said subject belonging to a certain class of subjects,
(b) a localization step, during which, by shape recognition, at least one portion of the vascular image to be studied (VI0) is automatically compared with a brain vascular atlas (VA) corresponding to said class of subjects, and the vascular image to be studied (VI0) is thereby located in the brain vascular atlas (VA),
(c) an identification step during which a brain functional atlas (FA) corresponding to said brain vascular atlas (VA) and comprising brain functional areas (1c) located in this brain vascular atlas (VA) is used, so that at least one brain functional area (1c) is identified on the vascular image to be studied (VI0).

2. The method according to claim 1, wherein at least during the localization step (b), a portion (VIR) of the brain vascular atlas (VA) is matched with the vascular image to be studied (VI0), and during the identification step, a map of functional areas (FI0) corresponding to said portion of the brain vascular atlas (VA) is determined, and the vascular image to be studied (VI0) is superposed with said map of functional areas (FI0).

3. The method according to claim 2, wherein, during the localization step, a geometrical deformation is determined giving the possibility of passing from said at least one portion (VIR) of the brain vascular atlas (FA) to the vascular image to be studied, and during the identification step, the functional map (FI0) of the vascular image to be studied is determined by applying this geometrical deformation to a portion (FIR) of the brain functional atlas (FA) corresponding to said portion (VIR) of the brain vascular atlas (VA) which corresponds to the vascular image to be studied (VI0).

4. The method according to any one of the claims 1 to 3, wherein in the localization step (b), said shape recognition is carried out by correlation between the vascular image to be studied (VI0) and the brain vascular atlas (VA).

5. The method according to any one of the claims 1 to 4, wherein the vascular image to be studied (VI0) includes at least one piece of hemodynamic information selected from among: the brain vascular volume, the Doppler power, the flow rate of the blood, the Doppler color, a value representative of the circulation resistance.

6. The method according to any one of the claims 1 to 5, wherein the brain vascular atlas contains data stemming from an imaging selected from among: ultrasonic imaging, angioscanner, MRI, CT scanner.

7. The method according to any one of the claims 1 to 6, further comprising, between the imaging step (a) and the localization step (b), a preliminary localization step (b0) during which on the vascular image to be studied (VI0), at least one characteristic area normally present in any subject to be studied and in the brain vascular atlas (VA) is automatically detected, and the vascular image to be studied (VI0) is thus roughly located in the brain vascular atlas (VA).

8. The method according to claim 7, wherein said characteristic area is selected from among: the Willis polygon, the Sylvian veins, the anterior brain artery, the largest arteries of the brain.

9. The method according to any one of the claims 1 to 8, including at least one preliminary step (p) for reference functional vascular mapping, comprising the following sub-steps:
(p1) a reference imaging sub-step during which are produced both:
- said brain vascular atlas (VA) of at least one subject from the class of considered subjects,
- and a reference anatomic atlas produced by imaging the brain of said at least one subject of the class of considered subjects, by a second type of imaging giving a more accurate anatomic image of the brain than ultrasonic imaging,
(p2) a reference functional mapping sub-step during which, said brain functional atlas (FA) is determined from a functional anatomic atlas which comprises at least one typical anatomic atlas of the brain and of the functional areas located on this typical anatomic atlas, this reference functional mapping sub-step being carried out by matching said reference anatomic atlas with the typical anatomic atlas, in order to locate the functional areas of the functional anatomic atlas on said brain vascular atlas.

10. The method according to claim 9, wherein at least during the reference imaging sub-step (p1), said brain vascular atlas (VA) is produced by vascular ultrasonic imaging of the brain.

11. The method according to claim 9 or claim 10, wherein during the reference functional mapping sub-step (p2), the reference anatomic atlas is matched with the typical anatomic atlas by shape recognition.

12. The method according to claim 11, wherein during the reference mapping sub-step (p2), a geometrical deformation is determined which gives the possibility of passing from said reference anatomic atlas to the typical anatomic atlas, this geometrical deformation is applied to the brain vascular atlas (VA) and the brain vascular atlas (VA) is thus matched with the functional areas of the functional anatomic atlas.

13. The method according to any one of the claims 9 to 12, wherein during said reference imaging sub-step (p1), respectively, several initial vascular images (VI) are successively carried out by vascular ultrasonic imaging of the brain and several initial anatomic images by said second type of imaging, are successively carried out on several subjects of the class of considered subjects, and said brain vascular atlas (VA) and said reference anatomic atlas are determined by a statistical calculation respectively from said initial vascular images and from initial anatomic images.

14. The method according to any one of the claims 1 to 13, wherein each class of subjects corresponds to at least one criterion selected from among: species, gender, age, weight.

15. A device for applying a method according to any one of the claims 1 to 14, comprising:
- vascular imaging organ (2-4) adapted for imaging a brain of a human or animal subject by ultrasonic imaging, in order to obtain a vascular image to be studied (VI0), said subject belonging to a certain class of subjects,
- localizing organ (4) by shape recognition, adapted for automatically comparing the vascular image to be studied (VI0), with a brain vascular atlas (VA) corresponding to said class of subjects, and for thereby locating the vascular image to be studied (VI0) in the brain vascular atlas (VA),
- identification organ (4) using a brain functional atlas (FA) corresponding to said brain vascular atlas (VA) and comprising brain functional areas (1c) located in this brain vascular atlas (VA), said identification organ being adapted for identifying at least one brain functional area (1c) on the vascular image to be studied (VI0).
